# EUROPEAN PATENT SPECIFICATION

(11) **EP 2 898 881 B1**
(45) Date of publication and mention of the grant of the patent: **10.10.2018**
(21) Application number: 15153105.0
(22) Date of filing: 01.08.2011
(51) Int. Cl.: A61K 31/132, A61K 31/724, A61P 15/02, A61K 9/00

(54) **Compositions comprising supramolecular complexes of polyanionic polymers and spermidine for use in the treatment of periodontum and damaged oral tissue**
Zusammensetzungen mit supramolekularen Komplexen von polyanionischen Polymeren und Spermidin zur Verwendung bei der Behandlung von Periodontium und beschädigtem Mundgewebe
Compositions comprenant des complexes supramoléculaires de polymères polyanioniques et spermidine destinés à être utilisés dans le traitement de tissus buccaux endommagés et du parodonte

(30) Priority: 04.08.2010 IT MI20101491; 14.12.2010 IT MI20102277; 16.12.2010 IT MI20102308
(43) Date of publication of application: 29.07.2015
(62) Divisional of application: 11814164.7
(73) Proprietor: PIERREL PHARMA S.R.L., 81043 Capua (CE) (IT)
(72) Inventor: Ghisalberti, Carlo, 04542-080 Sao Paulo (BR)
(74) Representative: Ripamonti, Enrico

(56) References cited:
- EP-A1- 1 407 785
- EP-A1- 1 884 231
- WO-A1-2007/104981
- WO-A1-2010/049562
- WO-A2-99/51213
- STABELLINI G ET AL: "Effects of spermine and spermidine on glycosaminoglyan metabolism in primary cultures of chick embryo fibroblasts", MEDICAL SCIENCE RESEARCH, ELSEVIER APPLIED SCIENCE, BARKING, GB, vol. 17, no. 21, 1 January 1989 (1989-01-01), pages 915-916, XP009098831, ISSN: 0269-8951
- GHISALBERTI CARLO A ET AL: "Potent trophic activity of spermidine supramolecular complexes in in vitro models.", WORLD JOURNAL OF BIOLOGICAL CHEMISTRY 26 AUG 2013, vol. 4, no. 3, 26 August 2013 (2013-08-26) , pages 71-78, XP002718871, ISSN: 1949-8454

## Description

### FIELD OF THE INVENTION

The invention refers to compositions comprising supramolecular complexes formed by polyanionic polymers and spermidine for use in the treatment, maintenance or repair of periodontum and oral tissues.

### BACKGROUND

Spermidine belongs to the group of polyamines (PA), metabolic polycations that link negatively charged DNA, RNA, proteins, phospholipids, and nucleoside triphosphates.

This ability could explain a contribution in cell proliferation and differentiation, see Heby O. Differentiation 1981;19:1-20; and Cohen SS. A Guide to the Polyamines. New York: Oxford University Press; 1998;185-230.

Nevertheless their biological role is difficult to be clearly defined. PA are in fact required for transcription of the proto-oncogenes c-myc and c-fos. In particular, spermidine preferentially stimulates the transcription and the expression of c-myc, while putrescine of c-fos (Tabib & Bachrach Int J Biochem Cell Biol 1999;31:1289-95). A role in transduction of signals between cell membrane and nucleus was previously found (Tabib & Bachrach. Biochem Biophys Res Commun 1994;202:720-7). Spermidine is also involved in TGF-β signal transduction (Blachowski S et al., Int J Biochem 1994;26:891-897) and seems to be necessary for normal expression of the TGF-β gene during cell migration.

Several patents claim spermidine and other PA in various therapeutic contexts, namely WO97/014415 and WO99/051213 for local analgesia and eczema; US6555140 to increase male fertility and libido; US06252838 as skin anti-aging; US04242701 to treat alcoholism; WO9852552 as anti-cancer; and US5432202 as anti-hypertensive Ca-antagonists.

A typical behaviour of spermidine is the interaction with anionic macromolecules to produce supramolecular complexes, which for example modulate the enzyme/DNA interactions (Isobe H et al. Chem Commun (Camb). 2005; 28;(12):1549-51).

Supramolecular complexes formed by spermidine and phosphate groups change the condensation status of DNA, and protect it from the nucleases activities (D'Agostino L et al. IUBMB Life. 2006;58(2):75-82). A similar property was found on the spermidine supramolecular, self-assembled aggregates of the polyamine reportedly behaving as protecting factors (D'Agostino L et al. FEBS J. 2009;276(8): 2324-35).

WO2010049562 discloses the supramolecular complexes of polyanionic polymer with spermidine or spermine by ratios comprised between 1:0.1 and 1:05 w/w, that clearly approach the equimolarity. Their purported use is the delivery of other bioactive agents.

The only known application are the anti-alopecia products marketed as Bioscalin® by Giuliani SpA (Milan, Italy), with spermidine HCl branded Biogenina and Cronobiogenina. Formulations and anti-alopecia methods based on spermidine HCl have been patented by Giuliani in EP1469843, WO03063851, and WO2010060729.

While there is a clear need for therapeutics capable to promote periodontum and oral tissue regeneration by means of cell proliferation, the design of spermidine complexes having high efficiency in this specific issue has never been attempted so far.

### SUMMARY

It was surprisingly discovered that certain supramolecular complexes formed by polyanionic polymers and spermidine have high efficiency in periodontum and oral connective regeneration.

In an aspect, the invention refers to compositions comprising supramolecular complexes characterized by ratios of anionic equivalents (of polyanionic polymer) and cationic equivalents (of spermidine) from 10:1 to 10⁷:1 eq/eq, further characterized by high efficacy in regenerative treatments.

In another aspect, the inventive supramolecular complexes have preferred ratios from 10²:1 to 10⁴:1 eq/eq.

In still another aspect, the invention refers to compositions comprising the afore said supramolecular complexes for the maintenance and repair of damaged or senescent periodontum and oral tissues.

These and other features of the invention are best described herein after.

### DETAILED DESCRIPTION

The present invention is defined in the appended claims.

It is a subject-matter of the disclosure a composition comprising a supramolecular complex formed by at least a polyanionic polymer and spermidine with a ratio of anionic equivalents and cationic equivalents (of spermidine) from 10:1 to 10⁷:1 eq/eq, where the components of said supramolecular complex are intimately admixed, without any covalent bond between them, said composition being for use in maintenance and repair of a damaged or senescent connective tissue selected from oral mucosa, gingiva and periodontum.

The expression "supramolecular complex" as used herein describes a polyacid/polybasic complex formed by polyanionic polymer(s) and spermidine characterized by a ratio of anionic eq. (from polyanionic polymers) and cationic eq. (from spermidine) from about 10:1 to about 10⁷:1 eq/eq, more preferably from about 10²:1 to about 10⁴:1 eq/eq.

These supramolecular complexes for use according to the invention possess a marked proliferative/regenerative activity, that is significantly superior to spermidine, the polyanionic polymer, and the sum thereof.

Said supramolecular complexes are characterized by a ratio ≥ 10:1 e/eq, in that differing from complexes of polyanionic polymer and spermidine approaching equimolarity both by their structural features and by a significantly higher proliferative activity on fibroblasts.

The expression "polyanionic polymer and spermidine approaching equimolarity" as used herein means complexes having ratios from about 1:3 to 10:1 eq/eq of anionic equivalents from the polyanionic polymer to cationic equivalents from spermidine.

The expression "polyanionic polymer" refers, in the broadest sense understood in the art, to a polymeric material or polymer comprising a plurality of several anionic moieties per molecule. It includes natural/semi-synthetic polymers, and fully synthetic polymers containing a plurality of anionic moieties such as carboxylic (-COO⁻), sulphate (-OSO₃⁻), sulfonate (-SO₃⁻), phosphate (-OPO₃²⁻), phosphonates (-PO₃²⁻), and combination thereof.

The expression "polyanionic polymer" includes "polyanionically-derivatised polymer", meaning previously non-polyanionic polymers being converted into polyanionic polymer with suitable derivatizating reactants. Exemples of derivatizations are carboxymethylation, succinylation, or maleylation for carboxy groups; sulfation/sulfonation/sulfinilation for sulfate/sulfonate groups; phosphation/phosphorylation for phosphate/phosphonate groups.

Polyanionic polymers include anionic phyto-polysaccharides, phyco-polysaccharides, and endopolysaccharides; semi-synthetic, and fully synthetic polyanionic polymers.

Natural polyanionic polymers may be phyto- and phyco-polysaccharides such as alginates, agar, gellan gum, ghatti gum, karaya gum, tragacanth gum, wellan gum, xanthan gum, κ- ι-, and λ-carrageenan, xylomannan sulfate, fucoidan, and fucogalactan.

Other natural polyanionic polymer may be the endopolysaccharides such as hyaluronate, cross-linked hyaluronate, and other glycosoaminoglycan like heparin, supersulfated and modified heparins, e.g. supersulfated heparin, fraxiparin, fondaparin, idraparin, chondroitin sulfate A, B, and C, and the K5 derivatives.

Suitable hyaluronate, alias hyaluranan (HA) may be either of animal or microbial origin, with molecular weight (MW) in ranging from 5,000 kDa to 10 MDa.

Semi-synthetic polyanionic polymers may be carboxymethylated polysaccharides such as carboxymethyl cellulose, carboxymethyl starch, carboxymethyl dextran, carboxymethyl chitosan, carboxymethyl chitins; sulphated polysaccharides such as rhamnan sulfate, dextran sulfate, cellulose sulfate, sulfochitosans, curdlan sulfate, glyloid sulfate (GP4324), carob gum sulfate (GP4327), pentosan polysulfate (PPS); and phosphated polysaccharides such as phosphocellulose, and phosphochitosan.

Semi-synthetic polyanionic polymer may also be HA derivatives, e.g. the Fidia HA derivatives; or the thiolated polysaccharides such as thiolated cellulose, thiolated alginates, thiolated chitosan, thiolated hyaluronate from ThioMatrix-Green River Polymers GmbH (Insbruck, Austria), and the like.

Synthetic polyanionic polymer may be polyacrylates and polymethacrilates, linear and cross-linked homopolymers and copolymers thereof such as acrylates/acrilamides, acrylates/alchyl C10-C30 acrylates, acrylates/octylacrylamides; Carbopol™, Carbomer™ and Pemulen™ from Noveon-Lubrizol, butyl-polyacrylic acid, poly(acrylate-co-acrylamidomethylpropane sulfonate), poly(acrylate-co-vinylsulfonate), and poly(acrylate-co-vinylbenzenesulfonate) copolymers; methylvinylethere/maleic and other maleate copolymers, *alias* "polymaleates", e.g. of Gantrez™ type (ISP Corp.); and furthers as per US2010172861 or US2003021793; as well as poly(sodium 4-styrene sulfonate), Y-ART-4, suramin, thiolated carbomers, thiolated poly(met)acrylic acid, and the like.

The expression "polyanionic polymer" also encompasses anionic inorganic polymers such as polyphosphates; or recombinant polymers as disclosed in WO2002/077036.

Preferred polyanionic polymers for the inventive purposes are linear and crosslinked hyaluronates, alginates, linear and crosslinked polyacrylates, and polymaleates.

The polyanionic polymer to be used according to this invention has a molecular weight ≥ 5000 Da; and/or an anionic density from 0.1 to 18 meq/g, preferably from 1 to 14 meq/g.

For the inventive purposes spermidine, N-(3-aminopropyl)-1,4-butanediamine, or "Spd" hereafter, is the substance of formula NH₂(CH₂)₃NH(CH₂)₄NH₂ as such or salt thereof may be used preferably of synthetic origin and, also preferably, of purity ≥ 99% on dry basis.

The supramolecular complexes for use according to the invention are produced by acid-base exchange, e.g. by combining a polyanionic polymer salt (typically Na) with spermidine salt (e.g. 3HCl); or by combining polyanionic polymers in the acid form with spermidine as free base.

The remaining anionic equivalents in the polyanionic polymer within the complex may be in acid form or partially neutralized, e.g. with alkaline or earth-alkaline ions such as Na, K, Li, Ca, and Mg; or amines such as NH₄⁺, mono- di- and triethanolamine, tromethamine, isopropylamine, lysine, etherocyclic amines such as piperazine, and the like.

The combination of polyanionic polymer with spermidine may be carried out in solvated status, preferably in water and/or water-soluble solvents such as lower alcohols, etc. The so-formed supramolecular complex may be dried up, e.g. by liophylization, spray-drying, vacuum drying, and the like; or used as such in solution form, provided that the solvents were physiologically acceptable and compatible with the desired end-use.

In an embodiment, supramolecular complex for use according to the invention are formed by combining one or more polyanionic polymer with spermidine directly within the tank-mixer used in the galenic manufacturing. It can be performed, e.g., by mixing the aqueous solutions of the reactants, optionally along with selected co-solvents, excipients, diluents, carriers, and adjuvants.

In alternative, the supramolecular complex for use according to the invention are prepared directly in solid state, e.g. by spraying a spermidine solution onto the polymer or, alternatively by blending or grinding the components in dry or partially wet forms, and the like procedures

Notwithstanding the applied procedure, the obtained supramolecular complexes for use according to the invention may be in admixture with several further excipients, diluents, carriers and adjuvants to produce a composition suitable for periodontum and oral tissue repair and maintenance.

In accordance with an aspect of the present invention, there are provided compositions and methods to regenerate connective periodontum and oral tissues by eliciting/enhancing cell proliferation.

The compositions for use according to the invention are administered to improve senescent periodontum and oral tissues or to repair damaged periodontum and oral tissues, including:
- oral mucosa, i.e. in stomatology, to treat stomatitis, aphthous ulcer, and xerostomia,
   dry mouth and/or Sjogren's syndrome;
- gingiva and periodontum, i.e. in periodontology, to treat gingivitis/periodontitis;

The compositions of invention will comprise supramolecular complexes of 10³-10⁷ eq/eq ratio in amount from about 0.01% to about 10% w/w; and supramolecular complexes of 10-10² eq/eq ratio from about 0.0001% to about 10% w/w of the composition.

A composition is preferably designed to provide an amount of spermidine from about 10 µmol to 0.1 nmol per unit dose, more preferably from about 1 µmol to 1 nmol per unit dose, even more preferably from about 100 nmol to 10 nmol per unit dose.

Low dosage levels are recommended in leave-in compositions, wherein a long period of time is sufficient to release a therapeutic amount of spermidine onto the target periodontum and oral tissue. The upper dosage levels are instead recommended in rinse-off or other compositions with short-time contact to provide a sufficient level of spermidine to elicit periodontum and oral tissue regeneration. The amount and duration of treatment shall be determined according to the target and patient condition, typically over a period from 30 to 60 days or more until relief is achieved, than it may be ceased, tapered, or reduced for an indefinite period.

Noteworthy, the composition for use according to the invention differs from compositions, if any, occasionally comprising polyanionc polymer(s) and spermidine that were separately admixed thereto and thus may not, or just partially do, produce the corresponding supramolecular complex.

The composition for use according to the invention may be produced according to known techniques with physiologically acceptable ingredients and carriers in order to afford the better benefit/risk profile, e.g. those listed in INCI-CTFA Annex 93/35/ECC and/or in Pharmacopoeias.

A fluid composition may have different presentations, including gel, lipogel, aerosol, spray, lotion, milk, foam, cream, W/O, O/W or multi-phase emulsions, mucoadhesive patches and so on, along with suitable excipients, carriers, or propellants.

The compositions for oral mucosae may be formulated in many ways, e.g., as per ADA/PDR: Guide to Dental Therapeutics, 4th Ed. such as mouthwash, oral solution, spray, gel on film, dentifrices, tooth powder, dental tablets, cream and gels, chewing gum, chewable tablets and lozenges, gel, film, gel-on-film, granules, paste, spreadable powders, etc. for application on oral cavity directly or by an external device.

### EXAMPLES

### EXAMPLE 1 - Metabolic pattern of HA-Spd 50:1 eq/eq in native gingival fibroblasts

Tissue specimens of non-inflamed periodontal gingival were obtained from the premolar area during oral surgery (six females, 20-30 years old). Each biopsy was washed with 0.1 M D-PBS and immediately minced with sterile scissors. Tissue fragments were transferred to 25 cm2 Nunc flasks and, after adherence, supplemented with 5 ml DMEM containing 10% BFS 100 UI/ml penicillin, 10 ng/ml streptomycin, and 25 µg/ml amphotericin B. Cultures were maintained in humidity saturated atmosphere (5% CO₂, 37°C) and routinely subcultured after use of 0.1% trypsin 0.02% EDTA for cell release. At given times cell culture supernatants were collected and fibroblasts washed in PBS, trypsinized and harvested by centrifugation (100 xg, 5 min). Treatment: 4 doses, 1 time point (24 or 48h) with sample treated with HA-Spd 10⁴:1, whilst untreated specimen served as control.

mRNA levels for TGF-β1, LH2B, TIMP-1, TIMP-2 and GAPDH by real-time RT-PCR Total RNA was isolated by a modification of the acid guanidinium thiocyanate-phenolchloroform method (Tri-Reagent, Sigma). 1 µg of total RNA was reverse-transcribed in 20 µl final volume of reaction mix (Biorad). The primers sequences for the target genes was Beacon Designer 6.0 Software (BioRad). GAPDH was used to normalize for differences in amount of total RNA in each sample. Amplification was conducted in a final volume of 20 µl per well with 10 µl of 1x SYBR Green Supermix (BioRad), 2 µl of template, 300 pmol of each primer, each sample analyzed in triplicate in duplicate amplifications. The cycle threshold and gene expression levels relative to GAPDH was calculated by 2^{-ΔΔCt} method.

COL-I and COL-III protein levels by slot blot. To asses both COL-I and COL-III secreted by palate and tuberosity fibroblasts, cell culture media was concentrated 20-fold with Centricon 10 columns (Amicon Y10, Millipore). Protein content was determined by a colorimetric assay (DC Protein Assay, Bio Rad); 100 µg of total protein per sample in final vol. of 200 µl of Tris buffer saline (TBS) was spotted onto a nitrocellulose membrane in a Bio-Dot SF apparatus (Bio-Rad). Membranes were blocked for 1 h with 5% skimmed milk in TBST (TBS containing 0.05% tween-20), pH 8, and incubated for 1 h at room temperature in monoclonal antibody to COL-I (1:1000 in TBST) (Sigma) or to COL-III (1:2000 in TBST) (Sigma). After washing, membranes were incubated in HRP-conjugated rabbit anti-mouse serum (1:80,000 in TBST) (Sigma) for 1 h. Immunoreactive bands were Amplified Opti-4CN substrate (Bio Rad) and scanned (UVBand, Eppendorf).

MMP-1 protein levels and activity by western blot. Concentrated culture media (5 µg of total proteins) was diluted in SDS-sample buffer, loaded on 10% SDS-polyacrylamide gel, separated under reducing and denaturing conditions at 80 V, and transferred at 90 V to a nitrocellulose membrane in 0.025 M Tris, 192 mM glycine, 20% methanol, pH 8.3. After electroblotting, the membranes were air dried and blocked for 1 h. After washing, membranes were incubated for 1 h at room T in monoclonal antibody to MMP-1 (1 µg/ml in TBST, Oncogene Research) and, after washing, in HRP-conjugated rabbit anti-mouse serum (1:40000 dilution, Sigma-Aldrich). Immunoreactive bands were revealed by Amplified Opti-4CN substrate (Bio Rad) and scanned (UVBand, Eppendorf).

Gelatinases (MMP-2 and MMP-9 activity) by SDS-zymography. Concentrated culture media was mixed 3:1 with sample buffer (containing 10% SDS). Samples (5 µg of total protein per sample) were run under non-reducing conditions without heat denaturation onto 10% SDS-PAGE co-polymerized with 1 mg/mL of type I gelatin. The gels were run at 4°C. After SDS-PAGE, the gels were washed twice in 2.5% Triton X-100 for 30' each, incubated overnight in a buffer at 37°C. The MMP gelatinolytic activity was detected after staining the gels with Coomassie brilliant blue R250, as clear bands on a blue background.

Fibroblast count The effect of proliferation was assessed by the cell ability to exclude trypan blue, performed according to Patterson MK Jr in: Jacob & Pastan, eds. Methods in Enzymology, vol. 58. New York: Academic Press; 1977:141.

Results reveal a complex pattern of regulatory activities of degradative enzymes such as collagenases and MMP; and modulation of TGF-β1, LH2B, TIMP-1/2 and GAPDH, which revert the previous finding of Gagliano N at al. J Periodontol 2005; 76:443-9.

### EXAMPLE 2 - Simple HA-Spd gel

10 g of sodium HA (MW 1,2 MDa) was dissolved in 800 ml of water until full hydration, then 10 ml of ImM Spd were added under stirring for 5' to afford a HA-Spd 10²:1 eq/eq complex. Then 0.9 g of benzyl alcohol were admixed and stirred, and the final volume completed to 1 liter with purified water. The gel was loaded in 60-ml PE tubes suitable for most applications as uro-genital, oral, ear, nose, throat mucosae, skin, and so on.

### EXAMPLE 3 - Polymaleate-Spd gel

A gel was prepared with the ingredients as set forth in Table I below.

**TABLE I**

| Ingredient | Quantity (in 100 ml) |
|---|---|
| Gantrez S97BF | 1.00 g |
| Spermidine 3HCl, 1mM | 100 µl |
| Propylen glycol | 15.0 g |
| Parabens | 0.50 g |
| Purified water | to 100 ml |

Gantrez S97BF, a polymaletate copolymer by ISP Corp (Wayne, NJ, USA) was dispersed in water and 8 ml 1N NaOH. Spermidine solution was added and stirred for 5' to afford polymaleate-Spd 3x10⁴:1 eq/eq complex. The other components are admixed to end up with a consistent gel suitable for most applications cited herein.

### EXAMPLE 4 - Mixed polyanionic-Spd gel

A gel was prepared with the ingredients as set forth in Table II below.

**TABLE II**

| Ingredient | Quantity (in 100 ml) |
|---|---|
| Sodium HA | 0.20 g |
| Sodium carboxymethylcellulose (CMC) | 2.50 g |
| Polycarbofil | 0.30 g |
| Spermidine 3HCl, 1mM | 2.50 ml |
| PEG-40 hydrogenated castor oil | 1.00 g |
| Disodium EDTA | 0.05 g |
| Xylitol | 7.50 g |
| Dichlorobenzyl alcohol | 0.50 g |
| Cu chlorophyll | 0.12 mg |
| Favours | 0.30 g |
| NaOH | to pH 6.5 |
| Purified water | to 100 ml |

Sodium HA, Blanose 7HXF (CMC), and Noveon AA-1 polycarbophil were dissolved in water until full hydration affording a viscous gel. The spermidine solution was then added and stirred for 15'. Remaining ingredients were singularly added and mixed to afford a homogenous green-coloured gel useful for mucosa repair.

### EXAMPLES 5-6 - Polymaleate-Spd gel with NAC and mucoadhesion inhibitor

A gel was prepared with the ingredients as set forth in Table III below.

**TABLE III**

| Ingredient | Quantity (in 100 ml) |
|---|---|
| Gantrez S97BF | 0.17 g |
| Spermidine 3HCl, 1mM | 500 µl |
| Polaxamer 427 | 20.0 g |
| PEG-40 hydrogenated castor oil | 1.00 g |
| Povidone | 5.00 g |
| Sodium saccharinate | 0.30 g |
| Benzalkonium chloride | 0.10 g |
| N-acetyl-cysteine (NAC) | 0.30 g |
| d-Mannose | 4.00 g |
| NaOH 1N | to pH 6 |
| Purified water | to 100 ml |

Polymaleate (Gantrez S97BF) was suspended in water and titrated with IN NaOH untill pH 6. Spermidine was then admixed to afford a polymaleate-Spd 10³:1 eq/eq complex.

The addition of next ingredients ended up with a homogeneous gel suitable for use on damaged mucosae having a ancillary biofilm disruptor activity.

An still enhanced formulation was conceived with the addition from 5% to 15% w/w of D-Mannose to afford the inhibition of bacterial adhesion onto the so-treated mucosae.

### EXAMPLE 7 - Carbopol-Spd modified mouthwash

1 g of Carbopol Ultrez 20 (Noveon-Lubrisol) was dissolved in 500 ml of the commercial mouthwash Iodosan Antiplacca (Iodosan SpA, GSK group). The pH was corrected to 6.5 with IN NaOH. Then 5 ml of ImM spermidine 3HCl were added and mixed until a homogeneous solution suitable for oral care, stomatology issues and gingivitis.

### EXAMPLE 8 - HA-Spd chewing-gum

A blend comprising xylitol, sodium HA and spermidine at 100:10:1 w/w/w ratio obtained by dry grinding said components was supplied to Gum Base Co Srl (Lainate, Italy) with the instruction to manufacture of chewing gum with 0.8 % of such blend. The resulting product is characterized by pleasant palatability usefully applied in gingival healing.

### EXAMPLE 9 - In vivo evaluation on stomatitis (case study)

A 46-years female with recurrent aphthous ulcer on mouth was given the gel of Example 21 and instructed to apply it at least twice a day. The subject referred a resolution in about a week against the 3-4 weeks generally needed. The product was well tolerated, except for the bitter taste, likely due to benzyl alcohol.

### EXAMPLE 10 - CMC-Spd gel dentifrice

A toothpaste was prepared with the ingredients as set forth in Table IV below.

**TABLE IV**

| Ingredient | Quantity (in 100 g) |
|---|---|
| Spermidine 3HCl, ImM | 2.00 ml |
| Sodium carboxymethyl cellulose (CMC) | 0.80 g |
| Xylitol | 1.00 g |
| Propylen glycol | 7.50 g |
| Sodium lauryl surcosinate | 1.70 g |
| Macrogol 1600 | 1.00 g |
| Aroma | 0.90 g |
| Sodium saccharinate | 0.20 g |
| Sodium phosphate | 0.20 g |
| Dibasic sodium phosphate 7H₂O | 0.45 g |
| Methylene blue, 1% | 0.10 g |
| Parabens | 0.20 g |
| Sorbitol, 70% | to 100 g |

The procedure follows the ordinary method for the production of a dentifrice, expect that CMC was first dissolved in water and added with the spermidine solution, then the remaining ingredients were incorporated to provide a blue translucent gel.

## Claims

1. A composition comprising a supramolecular complex formed by at least a polyanionic polymer and spermidine with a ratio of anionic equivalents to cationic equivalents from 10:1 to 10⁷:1 eq/eq, wherein the components of said supramolecular complex are intimately admixed, without any covalent bond between them, said composition being for use in stomatology to treat stomatitis, aphthous ulcer, and dry mouth, and in periodontology to treat gingivitis and periodontitis.

2. The composition for use according to claim 1, wherein said ratio is from 10²:1 to 10⁷:1 eq/eq, preferably from 10²:1 to 10⁴:1.

3. The composition for use according to claim 1, wherein the polyanionic polymers is a natural phytopolysaccharide, phycopolysaccharide, or endopolysaccharide; a semi-synthetic derivatized polysaccharide; or a synthetic polyanionic polymer, and mixture thereof.

4. The composition for use according to claim 3, wherein the phyto-, phyco- or endo-polysaccharide is selected from the group consisting of alginates, agar, gellan gum, ghatti gum, karaya gum, tragacanth gum, welan gum, xanthan gum, carrageenans, xylomannan sulfate, fucoidan, and fucogalactan, and linear or crosslinked hyaluronate.

5. The composition for use according to claim 3, wherein the semi-synthetic derivatized polysaccharide is selected from the group consisting of carboxymethyl cellulose, crosscaramellose, carboxymethyl starch, carboxymethyl dextran, carboxymethyl chitosan, linear or cross-linked hyaluronate, rhamnan sulfate, dextran sulfate, cellulose sulfate, curdlan sulfate, and phosphochitosan.

6. The composition for use according to claim 3, wherein the synthetic polyanionic polymer is selected from the group consisting of linear or crosslinked, homopolymer or copolymer acrylates and metacrylates, polycarbophil, Carbopol, maleic anhydride copolymers ("polymaleates"), and thiolated (poly)acrylates.

7. The composition for use according to any one of the preceding claims **characterized in that** it comprises supramolecular complexes of 10³-10⁷ eq/eq ratio in amount from about 0.01% to about 10% w/w of the composition.

8. The composition for use according to any one of claims 1 to 6, **characterized in that** it comprises supramolecular complexes of 10-10² eq/eq ratio from about 0.0001% to about 10% w/w of the composition.

9. The composition for use according to any one of the preceding claims **characterized in that** it is a mouthwash, an oral solution, a spray, a gel, a film, a dentifrice, a tooth powder, a dental tablet, a cream. a gel, a chewing gum, a chewable tablet or lozenge, a gel-on-film, granules, a paste, a spreadable powder.

## Patentansprüche

1. Zusammensetzung mit einem supramolekularen Komplex von mindestens einem polyanionischen Polymer und Spermidin in einem Verhältnis von anionischen Äquivalenten zu kationischen Äquivalenten von 10:1 bis 10⁷:1 Äq/Äq, worin die Komponenten des genannten Komplexes ohne kovalente Bindung dazwischen innig gemischt sind, wobei die genannte Zusammensetzung zur Verwendung in Stomatologie bei der Behandlung von Stomatitis, aphthösem Ulcus und Mundtrockenheit, sowie in Periodontologie bei der Behandlung von Gingivitis und Periodontitis ist.

2. Zusammensetzung zur Verwendung nach Anspruch 1, worin das Verhältnis von 10²:1 bis 10⁷:1 Äq/Äq, vorzugsweise von 10²:1 bis 10⁴:1 ist.

3. Zusammensetzung zur Verwendung nach Anspruch 1, worin das polyanionische Polymer ein natürliches Phytopolysaccharid, Phycopolysaccharid oder Endopolysaccharid; ein halbsynthetisches derivatisiertes Polysaccharid; oder ein synthetisches polyanionisches Polymer, sowie Mischung davon, ist.

4. Zusammensetzung zur Verwendung nach Anspruch 3, worin das Phytopolysaccharid, Phycopolysaccharid oder Endopolysaccharid aus einer aus Alginaten, Agar, Gellangummi, Ghattigummi, Karayagummi, Tragantgummi, Welangummi, Xanthangummi, Carrageenanen, Xylomannan-Sulfate, Fucoidan und Fucogalactan und linearem oder vernetztem Hyaluronat bestehenden Gruppe gewählt wird.

5. Zusammensetzung zur Verwendung nach Anspruch 3, worin das halbsynthetische derivatisierte Polysaccharid aus der aus Carboxymethylzellulose, Crosscaramellose, Carboxymethylstärke, Carboxymethyldextran, Carboxymethylchitosan, linearen oder vernetzten Hyaluronat-Derivaten, Rhamnansulfat, Dextransulfat, Zellulosesulfat, Curdlansulfat und Phosphochitosan bestehenden Gruppe gewählt wird.

6. Zusammensetzung zur Verwendung nach Anspruch 3, worin das synthetische polyanionische Polymer aus der aus linearen oder vernetzten Homopolymer- oder Copolymeracrylaten und -Methacrylaten, Polycarbophil, Carbopol, Maleinsäureanhydrid-Copolymeren ("Polymaleaten") und thiolierten (Poly)acrylaten bestehenden Gruppe gewählt wird.

7. Zusammensetzung zur Verwendung nach irgendeinem der vorangegangenen Ansprüche, **dadurch gekennzeichnet, dass** sie supramolekulare Komplexe in einem Verhältnis von 10³-10⁷ Äq/Äq in einer Menge von ungefähr 0,01 bis ungefähr 10 Gew-/% der Zusammenfassung umfasst.

8. Zusammensetzung zur Verwendung nach irgendeinem der Ansprüche 1 bis 6, **dadurch gekennzeichnet, dass** sie supramolekulare Komplexe in einem Verhältnis von 10-10² Äq/Äq in einer Menge von ungefähr 0,0001 bis ungefähr 10 Gew-/% der Zusammenfassung umfasst.

9. Zusammensetzung zur Verwendung nach irgendeinem der vorangegangenen Ansprüche, **dadurch gekennzeichnet, dass** sie eine Mundspülung, eine orale Lösung, ein Spray, ein Gel, eine Folie, ein Zahnputzmittel, ein Zahnpulver, eine Dentaltablette, eine Creme, eine Gl, einen Kaugummi, eine Kautablette oder -pastille, ein Gel auf Folie, Granulate, eine Paste, ein streichfähiges Pulver ist.

## Revendications

1. Composition comprenant un complexe supramoléculaire formé par un moins un polymère polyanionique et spermidine avec un rapport entre équivalents anioniques et équivalents cationiques entre 10:1 et 10⁷:1 eq/eq, où les composants dudit complexe supramoléculaires sont intimement mélangés, sans aucune liaison covalente entre eux, ladite composition étant destinée à être utilisée en stomatologie dans le traitement de stomatite, ulcère aphteux et sécheresse buccale, aussi bien qu'en parodontologie dans le traitement de gingivite et parodontite.

2. Composition destinée à être utilisée selon la revendication 1, où ledit rapport est compris entre 10²:1 et 10⁷:1 eq/eq, préférablement entre 10²:1 et 10⁴:1.

3. Composition destinée à être utilisée selon la revendication 1, où le polymère polyanionique est un phytopolysaccharide, phycopolysaccharide ou endopolysaccharide naturel ; un polysaccharide dérivatisé semi-synthétique ; ou un polymère polyanionique synthétique, et des mélanges des mêmes.

4. Composition destinée à être utilisée selon la revendication 3, où le phytopolysaccharide, phycopolysaccharide ou endopolysaccharide est choisi dans le groupe formé par alginates, agar, gomme gellane, gomme ghatti, gomme karaya, gomme tragacanthe, gomme welan, gomme xanthane, carraghénanes, xylomannane sulfaté, fucoidane et fucogalactane et hyaluronate linéaire ou réticulé.

5. Composition destinée à être utilisée selon la revendication 3, où le polysaccharide dérivatisé semi-synthétique est choisi dans le groupe formé par carboxyméthylcellulose, crosscaramellose, carboxyméthylamidon, carboxyméthyldextrane, carboxyméthylchitosane, dérivés de hyaluronate linéaire ou réticulé, sulfate de rhamnane, sulfate de dextrane, sulfate de cellulose, sulfate de curdlane et phosphochitosane.

6. Composition destinée à être utilisée selon la revendication 3, où le polymère polyanionique synthétique est choisi dans le groupe formé par acrylates et méthacrylates d'homopolymère ou copolymère linéaire ou réticulé, polycarbophile, Carbopol, copolymères d'anhydride maléique ("polymaléates") et (poly)acrylates thiolés.

7. Composition destinée à être utilisée selon l'une quelconque des revendications précédentes, **caractérisée en ce qu'**elle comprend des complexes supramoléculaires avec un rapport 10³-10⁷ eq/eq dans une quantité comprise entre approximativement 0,01 % et approximativement 10 % p/p de la composition.

8. Composition destinée à être utilisée selon l'une quelconque des revendications 1 à 6, **caractérisée en ce qu'**elle comprend des complexes supramoléculaires avec un rapport 10-10² eq/eq dans une quantité comprise entre approximativement 0,0001 % et approximativement 10 % p/p de la composition.

9. Composition destinée à être utilisée selon l'une quelconque des revendications précédentes, **caractérisée en ce qu'**elle est un bain de bouche, une solution orale, un spray, un gel, une pellicule, un dentifrice, un dentifrice en poudre, un comprimé dentaire, une crème, un gel, une gomme à mâcher, un comprimé ou une pastille à mâcher, un gel sur pellicule, des granules, une pâte, une poudre à étaler.
